# EUROPEAN PATENT APPLICATION

(11) **EP 4 563 141 A1**
(43) Date of publication of application: **04.06.2025**
(21) Application number: 24216864.9
(22) Date of filing: 02.12.2024
(51) Int. Cl.: A61K 9/00, A61K 8/00, A61K 9/107, A61K 31/00, A61K 47/10, A61K 47/12, A61K 47/44

(54) **SELF-EMULSIFYING FORMULATION FOR TOPICAL USE**

(30) Priority: 01.12.2023 IT 202300025656
(71) Applicant: Guidotti, Elena, 46051 San Giorgio Bigarello (MN) (IT); Lonati, Gisella, 46010 Curtatone (MN) (IT)
(72) Inventor: Guidotti, Elena, 46051 San Giorgio Bigarello (MN) (IT); Lonati, Gisella, 46010 Curtatone (MN) (IT)
(74) Representative: Bottino, Giovanni

(57) **Abstract**

Self-emulsifying formulation for topical, and typically skin use, comprising at least one highly lipophilic active component, a lipophilic portion, an amphiphilic portion, and a skin penetration-promoting portion derived from essential oils.

## Description

The present invention relates to formulations for pharmaceutical and/or cosmetic use, and specifically relates to self-emulsifying formulations for the release of active substances. More specifically, the invention relates to a self-emulsifying formulation for topical, and typically cutaneous, use.

Numerous formulations are known at the state of the art, made to make available to metabolism those substances that are poorly water-soluble, and thus are difficult to assimilate in the first digestive tract. These formulations, commonly known as SEDDS (an acronym for Self-Emulsifying Drug Delivery System, or self-emulsifying active delivery systems) must be designed in such a way as to balance the lipophilic character of the active ingredients that are to be delivered against the essentially hydrophilic environment in which their metabolism takes place instead.

If, on the other hand, the formulation is to be administered topically, the different factors that need to be balanced must take into account the different features of the layers that make up the epidermis, which exhibit equally different behaviors and affinities.

Topical administration inherently avoids the hepatic first-pass effect without the need for high surfactant content. The skin exhibits a highly accessible route for the administration of active compounds, mainly because hepatic metabolism is avoided with improved bioavailability of compounds, reduced adverse effects of some drugs, and improved patient compliance. However, despite the ease of application, as well as the accessibility of this multidimensional organ, the multilayered nature of the skin presents some challenges to the release of actives due to the lipophilicity of the outer stratum corneum, followed by the hydrophilicity of the underlying skin layers. Therefore, exploiting the sandwich mechanism of the inter-corneocyte cement and using nonionic amphiphilic surfactants of cement-like nature, spontaneous emulsion is sought as a topical system for potential transdermal absorption of actives.

The skin is the first barrier between the body and exogenous substances. In addition, the multilayered structure of the skin allows this organ to be a regulator of current signals by active metabolic pathways to influence physiological activities and to regulate homeostasis.

Therefore, the skin should be considered a metabolically active part of the human body, as the enzyme profile of the skin is quite similar to the enzyme profile of the liver. Skin enzyme activity is intensified within the deeper layers of the skin, such as the inner layers of the epidermis and the dermis, compared with the outer stratum corneum (SC). Metabolic studies have shown that skin enzyme activity can add up to about 10% of total liver metabolism. Thus, dermal administration can reduce but not eliminate the kinetics that the body applies to the xenobiotic. The process of skin metabolism is defined as a two-phase reaction, in which phase I consists of reactions e.g., oxidation, hydrolysis, reduction, and phase II refers to conjugate reactions.

Enzymes responsible for determining skin metabolism include cytochrome P450, nonspecific esterases, the flavin monooxygenase and transferase enzyme family. Therefore, active molecules that are able to cross the highly lipophilic SC still have to endure metabolic degradation after entering the underlying skin layers. There are three different pathways of transcutaneous penetration through which bioactive components can cross the SC stratum corneum, namely, the transcellular, transfollicular, and intercellular pathways. Hydrophilic keratin surrounds corneocytes, which are known as the main cells of the stratum corneum of the epidermis. Therefore, hydrophilic drugs tend to pass through the transcellular pathway provided by keratins especially when highly hydrated. Similarly, intercellular permeation generally is the favorable pathway for lipophilic drugs, which diffuse into the lipophilic, and actually amphiphilic, inter-corneocyte cement.

The most modest transcutaneous transport of actives is the trans-follicular pathway, where just 0.1 percent of the total skin surface area is covered by hair follicles and sebaceous glands. However, the lipid-rich sebum that fills the sebaceous glands could provide a potential pathway favored by the lipophilic nature of sebum. Sweat could be an obstacle in terms of diffusion of the lipophilic molecule due to its hydrophilic nature.

However, topically applied SEDDS can emulsify with the hydrophilic portion of sweat, reduce the hydrophilic barrier, emulsify with the hydrolipidic skin mantle, and achieve the right compromise between hydrophilicity and lipophilicity that allows the best diffusion coefficient across the skin barrier. The additional barrier provided by sweat could be nullified as an agent that slows skin permeation and, instead of being a mediator that prevents skin permeation, come to promote it, once incorporated into the emulsion with SEDDS.

Furthermore, it is a well-known fact that drugs and molecules suitable for transdermal topical administration must possess an amphiphilic nature. Those with Log P < 1 or > 4 have poor penetrative ability. Amphiphilic molecules that are neither too hydrophilic nor too lipophilic are favored. Amphiphilic molecules possess sufficient lipophilic qualities to cross the lipophilic outer skin layer and sufficient hydrophilic qualities to diffuse into the hydrophilic epidermis and dermis. Not all molecules of interest possess these intrinsic qualities, so by obviating this stumbling block with SEDDSs, it is possible to increase the proportion adsorbed through the skin of molecules that by themselves, might have much lower transcutaneous bioavailability.

Another potential approach of using topical/transdermal drugs may be to use the lipophilic nature of SEDDSs to reach the lymph nodes located within the epidermis and dermis in order to further refine the targeting of dermal delivery systems of actives. Lymphatic absorption of oral administration of SEDDSs demonstrated successful circumvention of hepatic first-pass metabolism resulting in increased bioavailability.

Topical/transdermal administration of molecules allows the transport of sufficient concentrations of actives prone to hepatic metabolism, such as cannabidiol, because passage through the liver is avoided during dermal administration of actives. Therefore, lower concentrations of actives incorporated into topical transdermal SEDDS may not pose a problem.

On the other hand, vehicles intended for topical/transdermal administration have high success rates due to improved flow values such as the high concentration of active in the vehicle that establishes a driving force for the active toward skin compartmentalization due to the concentration of active maintained on the skin surface. In addition, topical application also allows the active to be delivered for strictly local action, with reduced systemic exposure to some molecules.

It is therefore the aim of the present invention to provide a self-emulsifying formulation for the release of active substances, which is able of promoting skin-level absorption of highly lipophilic active substances, such as cannabis-derived substances, without causing damage or sensitizing the skin itself to any extent, and on the contrary ensuring high compatibility with all components of the epidermis.

Thus, the object of the present invention is a self-emulsifying formulation for skin use, comprising a highly lipophilic active component capable of interacting with cannabinoid and vanilloid receptors, a lipophilic portion, an amphiphilic portion, and a skin penetration-promoting portion derived from essential oils.

The active component includes at least one substance chosen from the group consisting of cannabidiol, capsaicin, piperine, palmitoylethanolamide, nerolidol, myrcene, β-caryophyllene, borneol, α-pinene, honokiol, magnolol, acetaminophen, phosphatidylcholine, or a mixture of two or more of the said substances. The active component is included in the formulation in a percentage that varies according to the claimed effect from 0.1 % to 35 %, where skin toxicity allows.

In a preferred embodiment, the lipophilic portion comprises unsaturated fatty acids, and preferably oleic acid and/or linoleic acid, in combination with medium-chain triglycerides; the latter are esters of glycerol with saturated fatty acids of 6 to 12 carbon atoms, such as caprinic, caprylic, and lauric acids; in an executive variant, the lipophilic portion also comprises esters of saturated fatty acids, such as isopropylpalmitate and isopropylmyristate. In a further variant, the lipophilic portion includes esters of benzoic acid with a C₁₂-C₁₅ alcohol, and tocopherol.

The amphiphilic portion comprises nonionic surfactants, such as polysorbates, and in particular polysorbate 80, in combination with suitable co-surfactants to reduce the interfacial tension of the formulation while decreasing the surfactant concentration. Among co-surfactants are preferred oleylpolyoxyglycerides, and propylene glycol monocaprylate.

The skin penetration-promoting portion derived from essential oils may include eucalyptus oil, and in particular includes eucalyptol; in addition, limonene, linalyl acetate, bisabolol are preferred among other terpenes.

In an embodiment, the formulation comprises as lipophilic portion a mixture of natural oils including high oleic olive oil, avocado oil, linseed oil, with medium chain triglycerides derived from coconut, in a percentage ranging from 5 to 60 percent; as the amphiphilic portion, a mixture of surfactants including polysorbate, oleyl polyoxyglycerides, and with the possible presence of the co-emulsifier propylene glycol monocaprylate, in concentrations varying from 5 to 45% depending on the concentration and Log P of the active or mixture of actives. As penetration promoters: limonene and eucalyptus, in concentrations ranging from 1 to 20%.

In another embodiment, the formulation comprises as the lipophilic portion a mixture of natural oils including high oleic olive oil and avocado oil, safflower oil, with medium chain triglycerides derived from coconut, in a percentage ranging from 5 to 40 percent; as the amphiphilic portion a mixture of surfactants polysorbate, oleyl polyoxyglycerides and with the possible presence of the coemulsifier propylene glycol monocaprylate, in concentrations varying from 5 to 55% depending on the concentration and Log P of the active or mixture of actives. As a penetration promoter: limonene in concentrations ranging from 1 to 20%.

In a further embodiment, the lipophilic portion comprises a mixture of natural oils including high oleic olive oil, avocado oil, and linseed oil, with medium chain triglycerides derived from coconut, in a percentage ranging from 5 to 40 percent; the amphiphilic portion comprises a mixture of surfactants including polysorbate, Caprilocaproyl polyoxyglycerides, and with the possible presence of the coemulsifier propylene glycol monocaprylate, in concentrations varying from 5 to 45% depending on the concentration and Log P of the active or mixture of actives. As penetration promoters: limonene and eucalyptus in concentrations ranging from 3 to 20%.

In an even further embodiment, the formulation comprises as a lipophilic portion a mixture of natural oils including high oleic olive oil, avocado oil, and linseed oil, with medium chain triglycerides derived from coconut, in a percentage ranging from 5 to 50 percent; as the amphiphilic portion, a mixture of surfactants including polysorbate, Lauroyl polyoxyglycerides, and with the possible presence as a coemulsifier of propylene glycol monocaprylate, in concentrations varying from 5 to 55% depending on the concentration and Log P of the active or mixture of actives. As penetration promoters: limonene and eucalyptus in concentrations ranging from 3 to 30%.

In all the above formulations, the coemulsifier may be ethoxydiglycol.

Another object of the present invention is the therapeutic use of a formulation of the type described above for the treatment of the human or animal body. Specifically, the therapeutic use relates to the treatment of conditions such as fibromyalgia, painful scar, joint pain from peripheral nerve injury, cutaneous allodynia, somatic pain point.

A further object of the present invention is the cosmetic use of a formulation of the type described above for the treatment of the human or animal body.

Further advantages and features of the formulation according to the present invention will be evident from the following.

Regarding the transcutaneous absorption of actives, the saturation level of oil phase triglycerides (especially SEDDS) appears to be a determining factor for bioavailability, as numerous studies have indicated that unsaturated fatty acids (UFAs) show more powerful penetration effects through the skin than saturated fatty acids (SFAs). This finding may be explained by the reduced ability of SFAs to dissolve within the natural lipids of the stratum corneum, leading to reduced lipid alteration of the SC and, subsequently, less effective penetration through the skin.

Therefore, considering the delivery of topical/transdermal actives, the choice of the oil phase is not only critical to solubilizing the lipophilic molecules, but also fundamental to facilitate improved penetration through the skin, as determined by the fatty acids that are part of the oil phase.

In the formulation according to the present invention, an unsaturated high oleic oil was used in the lipophilic portion, which aids solubilization and penetration of the actives. Oleic acid is the most recognized skin penetration promoter of all time. It is a typical component of natural oils that promote skin penetration, such as olive, avocado and Pequi oil. The mechanism of skin penetration enhancement, achieved by oleic acid, is partially due to the disorienting effect of lipids in the inter-corneocithal cement of the stratum corneum. In addition, oleic acid has greater ability to facilitate fluidization of SC lipids, which leads to increased permeation of the active in addition to increased flow values.

The fluidization theory is confirmed by observed studies on oleic acid in separate pools of stratum corneum lipid domains. Since this is a cosmetic skin application, the choice of oil phase must be appropriate to promote transcutaneous penetration and not injure the skin structure, thus causing no harm. Interestingly, oils rich in linoleic acid have shown skin repair capabilities by preventing water loss from the stratum corneum. They actually decrease TEWL.

These enhanced hydration properties, facilitated by linoleic acid, may also be typical of other fatty acids, particularly those of a saturated nature. For example, stearic acid is known for its high melting point and subsequent film formation on the skin surface. Stearic acid applied to the skin cannot split in the SC to achieve a lipid-displacing effect, but it facilitates the formation of a film that can contribute to improved skin permeation due to occlusive effects that cause increased hydration followed by loosening of the rigid corneous structure that allows permeation of actives into the underlying skin layers. Therefore, the crucial contribution of different fatty acids on the skin and, in particular, the potential influence of different fatty acids used in combination in order to achieve optimized topical/transdermal passage of actives is highlighted. Furthermore, it has been shown that polyunsaturated fatty acids (PUFAs) tend to participate in oxidative reactions to a greater degree than monounsaturated fatty acids (MUFAs). Thus, fatty acid composition may consequently influence the shelf life of topical/transdermal SEDDS.

In the lipophilic portion of the formulation according to the present invention, unsaturated fatty acids such as linoleic from flaxseed oil, and saturated fatty acids such as medium-chain triglycerides are present to achieve a solubilizing effect of the actives, promoting penetration, facilitating passage from the vehicle to the skin, and not injuring the skin structure.

The interfacial tension between the lipophilic components of SEDDSs and the hydrophilic components of gastric fluids for oral formulations, or sweat or TEWL for topical ones, are reduced by the addition of surfactants. They create an interfacial film between the two immiscible phases for the purpose of generating dispersion, which allows greater bioavailability.

Overall, nonionic surfactants are mainly used during the formulation of oral SEDDS, as these surfactants have a reduced toxicity profile compared with anionic and cationic surfactants. As the skin structure is different from the gastrointestinal one, with significantly less surface aqueous fluids than the gastric one, and as surfactants are components with skin irritant capacity if not well balanced, the balance between enhancer strength of the formulation, emulsifying strength and NON-toxicity to the skin, is crucial.

During the development of SEDDSs intended for the topical/transdermal route of molecules, the challenge is to find appropriate surfactant concentrations, as the skin is sensitive to surfactant concentrations. Skin irritation reactions associated with exposure with surfactants are related ability of surfactants to solubilize lipid membranes. However, this property of surfactants may contribute to an increased ability of molecules to pass through the dermis, as the stratum corneum is composed of lipids that form a formidable barrier to transdermal delivery systems. Thus, when deciding about surfactant concentrations in SEDDS intended for cutaneous use, the choice must be based on the principle of finding an area of compromise so as to not only include surfactant concentrations low enough to avoid irritation, but these concentrations must be high enough to achieve improved penetration through the skin.

The surfactants used are all nonionic in nature, known and used for a long time, so that their toxicity profile is known. Preferably, a polysorbate is used, the use of which is widespread in pharmaceuticals as well as cosmetics. Polysorbates are known and used because of the melting temperature of the actives. For topical application, they are used to increase the water retention capacity of the formulation. In addition, they are known to improve the oral bioavailability of drugs that are substrates of the P-glycoprotein.

Polysorbates are widely approved as emulsifiers, antifoaming agents, synthetic flavoring agents, stabilizers, and thickeners in foods, cosmetics, and pharmaceuticals. Because of their safety profile, they also find use in ophthalmic and parenteral products. Polysorbates are widely used in cosmetics, foods, and topical pharmaceutical formulations; they are generally regarded as nontoxic and nonirritating. They have shown low toxicity for both oral and topical administration, both subchronic and chronic. Two additional surfactants with co-surfactant functionality have been added to improve emulsifying and penetrative capacity.

Co-tensioactives are included in SEDDS formulations to achieve an additional decrease in interfacial tension between the two immiscible liquids by decreasing the surfactant concentration. This allows greater flexibility of the interfacial film. By conferring greater flexibility at the interface, the ease of emulsion production increases, droplet formation is made spontaneous, with smaller amounts of actual surfactants.

A first co-tensioactive is part of the category of oleylpolyoxyglycerides with self-emulsifying and solubilizing properties in both oral and topical pharmaceutical formulations. They are also authorized excipients in cosmetics and food products. They are water-dispersible co-surfactants useful for solubilizing and increasing the bioavailability of highly lipophilic molecules. In particular, this co-surfactant has a skin safety profile, as it can be used at concentrations up to 4 times higher topically and up to 90 percent in oral formulations.

In order to optimize the penetrative ability of the actives, and to keep the relative amounts of the other surfactants as low as possible, a third solubilizer was added: propylene glycol monocaprylate. It is used in lotions and creams for topical applications, where it serves as a solubilizer, water-in-oil emulsifier, and stabilizer for emulsions. It possesses emollient properties. Also used orally to increase solubility and bioavailability, and as an insoluble surfactant for forming auto-emulsions.

It is a priority during topical/transdermal administration of the molecule to increase the contact time between the skin surface and the applied formulation to improve skin diffusion of the actives. Thus, orally administered SEDDSs should exhibit rapid self-emulsion, while topical SEDDSs should not achieve rapid emulsion, as this implies a superior ability to resist exogenous water exposure. If a SEDDS is able to limit rapid emulsification, once exposed to exogenous water sources or sweat secretion, the formulation demonstrates greater occlusivity, followed by a greater opportunity for diffusion of the active through the skin.

With the aim of obtaining a topical SEDDS with safe and prolonged use as required by Regulation 1223/2009 for cosmetic use, the balance between surfactants, co-surfactants and penetration promoters was sought in order to obtain formulations capable of conveying actives such as synthetically or naturally derived CBD, PEA (palmitoylethanolamide) and some terpenes functional for cosmetic and even in the case, therapeutic activities, which are poorly absorbed by the skin given their high lipophilicity. Oils, including essential oils and their constituents, are widely known to have penetration-promoting activities and have been incorporated into a wide range of pharmaceutical formulations.

The skin is a complicated organ that protects the human body from its environment and maintains its hydration under healthy and pathological conditions. The outermost layer of human skin, the stratum corneum, is the main barrier to the permeation of xenobiotics through the skin. Therefore, many studies have focused on identifying chemical penetration enhancers to help penetrate this layer and target actives to the underlying layers and even, if targeted, to the systemic circulation.

Skin safety concerns have limited the use of many chemicals with transcutaneous penetration enhancing effects. Essential oils and their terpene constituents have been identified as a candidate group with promising potential to be clinically useful as penetration enhancers. Essential oils are natural oily liquids containing a mixture of volatile compounds found in different parts of plants, such as flowers, fruits, leaves and roots. They are composed mainly of monoterpenes, sesquiterpenes, carbohydrates, alcohols, ethers, aldehydes and ketones and are extracted in small quantities using techniques such as steam current and hydro-distillation.

Essential oils and their terpene components have been reported as promoters of successful penetration of various drugs through the skin, and many demonstrate potential for both hydrophilic and hydrophobic drugs. The basic mechanism responsible for the promoter activity of individual essential oils is unclear, and several mechanisms of action are proposed to be used based on (1) disintegration of the highly ordered intercellular lipid structure between corneocytes in the stratum corneum, (2) interaction with intercellular domains of proteins, which induces their conformational modification, and (3) increase in the coefficient of a molecule.

Plant oils are natural fixed oils pressed mainly from seeds and are a common constituent of pharmaceutical formulations used topically. They are a mixture of heterogeneous lipids composed mainly of triglycerides and a smaller concentration of components such as free fatty acids, mono- and di-glycerides, sterols, phosphatides, fatty alcohols, and liposoluble vitamins. Despite the differences between such oils and essential oils, particularly in volatility, physicochemical properties, and aromatic characteristics, natural oils play an important role in pharmaceutical formulations. They have safe profiles and common uses as emulsifying agents, stabilizing agents, and diluents for essential oils prior to topical application.

In addition, studies have indicated that they can impart an effect of actually increasing skin permeability. Free fatty acids (saturated and unsaturated) are the key components of plant oils. They modify the skin barrier reversibly by fluidizing and disintegrating stratum corneum lipids. In general, oils containing high percentages of unsaturated free fatty acids have been shown to have a more significant skin permeation-enhancing effect than those with saturated free fatty acids. Natural oils containing fatty acids have been reported by many studies to be effective enhancers of transdermal penetration for both hydrophilic and lipophilic drugs. Such oils include olive oil, almond oil, and jojoba oil.

Terpenes are simple hydrocarbon compounds of natural origin isolated from various sources such as plants and animals. The penetration enhancing activity of terpenes has greatly increased their use in pharmaceutical products. Generally, terpenes are clinically accepted and safely used as penetration enhancers for both hydrophilic and lipophilic drugs. One safe terpene-rich essential oil is Eucalyptus oil. Eucalyptus [Eucalyptus globulus] is a plant used in traditional medicine belonging to the Myrtaceae family. It is extracted by steam distillation from the leaves of this plant, the eucalyptus plant being a major source of essential oils with various biological activities. The main pharmaceutical component of eucalyptus oil is eucalyptol [1,8-cineole]. Eucalyptol, a monoterpene monocyclic ether, represents 70-90% of the content of eucalyptus oil, which also contains minor constituents such as α-pinene, cuminylaldehyde, limonene, α-fellandrene, p-cymene, trans-pinocarveol, and terpinen-4-ol.

The oil has antimicrobial activities, including antibacterial activity against Staph. aureus, antifungal and antiviral. It has promising potential for treating microbial infections, such as those encountered during wound healing, burns and herpes infections. It also has anti-inflammatory activity that helps relieve pain and rheumatoid arthritis. Evidence of antioxidant activity has also been reported. In addition, many studies have reported a potential role for eucalyptus oil in the formulation of topical preparations as a result of its ability to increase transcutaneous permeation.

Eucalyptus oil has been identified as the most popular essential oil used as a penetration enhancer in dermal and transdermal formulations. In addition, studies have reported its superior strength and effectiveness compared with other terpenes and essential oils containing fatty acids. However, studies have confirmed that eucalyptus oil shows weaker permeation activity than Azone^{®} (1-dodecylazacycloheptan-2-one), oleic acid, and isopropyl myristate. This permeation property is the reason for its frequent inclusion in formulations targeting deeper layers of the skin, such as targets of transdermal patches, vesicular systems, microemulsions, and nanoemulsions.

Eucalyptol is a colorless organic liquid with a camphor-like smell. It has a spicy taste with a cool sensation. Topical use of eucalyptol produces analgesia with a reduction in inflammation. It has been used as a penetration enhancer in pharmaceutical formulations of dermal and transdermal preparations, with ability to improve permeation of both hydrophilic and lipophilic drugs. It is a more effective penetration enhancer than fatty acids and essential oils containing fatty acids. It has been shown to increase the rate of skin permeation more than olive oil, linseed oil and sunflower oil, oleic acid and medium-chain triglycerides, but not limonene. It can be used alone or in combination with other penetration promoters in order to target molecules in the deeper layers of the skin. Balancing the quality and quantity of terpenes as penetration promoters is critical in order not to exceed their individual doses for skin and target organ safety for the adsorbed portion that manages to enter the systemic circulation. Each penetration promoter works best for certain molecules to be delivered, and finding the right balance allows the result to be achieved without causing irritation, thus making the product safe.

Limonene has an aliphatic hydrocarbon structure belonging to the monocyclic terpene class, and is found in more than 300 plants and fruits, including grapefruit, lemons and oranges. Limonene is a colorless aromatic liquid with a citrus odor. Limonene has been reported to improve the permeation of topically administered drugs. In addition, it has been reported to have therapeutic effects such as hypoglycemic, antitumor, antihypertensive, lipid-lowering and anti-inflammatory effects as well as antioxidant activity.

Limonene has a broad spectrum of permeation-enhancing activity with a greater ability to improve the penetration of hydrophilic and lipophilic drugs than some terpenes such as cineole and essential oils containing fatty acids, including olive oil. In addition, limonene has been successfully incorporated into transdermal [matrix and reservoir] patch formulations in order to deliver actives to the deeper layers of the skin.

Linalyl acetate exerts analgesic effects by inhibiting the nociceptive receptor TRPA1 (study in mice), so it integrates with other terpenes. The TRPA1 receptor, originally called ANKTM1 is a molecule conserved in several species during evolution; TRPA1 is a cationic channel that works as a cellular sensor, sensing mechanical, chemical and thermal stimuli, being a component of neuronal, epithelial, blood and smooth muscle tissues. In mammals, TRPA1 is largely expressed in primary sensory neurons that mediate somatosensory processes and nociceptive transmission. Recent studies have described the role of TRPA1 in inflammatory and neuropathic pain. Several studies evaluate the relevance of TRPA1 activation and blockade in pain transmission, as well as the mechanisms underlying its activation and modulation by exogenous and endogenous stimuli. The entourage effect of terpenes points to modulation of the cutaneous vanilloid and cannabinoid systems.

Bisabolol is one of the important monocyclic sesquiterpenes naturally derived from essential oils of many edible and ornamental plants. It was first obtained from Matricaria chamomilla, commonly known as chamomile or German chamomile. Available literature indicates that this plant along with other α-bisabolol-containing plants is commonly used in traditional medicine for potential health and general well-being benefits. Nutritional studies are indicative of the health benefits of α-bisabolol. Numerous experimental studies have demonstrated pharmacological properties of α-bisabolol, including anticancer, antinociceptive, neuroprotective, cardioprotective, and antimicrobial. It is known for its anti-inflammatory action. Less well known is its peripheral anti-nociceptive action by action on the NO-cGMP-K+ channel.

The successful incorporation of terpenes into advanced dermal/transdermal formulations such as SEDDS, nanoemulsions, microemulsions, vesicular systems, and transdermal patches makes them more attractive than oils containing fatty acids. Consequently, they are suggested as first-choice activators to increase transcutaneous penetration for topical formulations. Terpenes offer promising potential for permeation of hydrophilic and lipophilic drugs through/in the skin using different mechanisms and are likely to play a significant role in the development of effective dermal and transdermal formulation.

Selection of the best terpene is an important factor to consider. Despite the similarity in the chemical structure of terpene-type essential oils, there is a difference in the mechanisms of enhancing the permeation of molecules through/into the skin. Influencing the selection of specific terpenes commonly reported in the formulation of some topical formulations means changing safety and efficacy.

The active substance of the formulation according to the present invention comprises a substance chosen from the group including cannabidiol, capsaicin, piperine, palmitoylethanolamide, nerolidol, myrcene, β-caryophyllene, borneol, α-pinene, honokiol, magnolol, acetaminophen, and phosphatidylcholines, either alone or in mixture with each other. The main properties related to these compounds, which determined the choice of using them in the active substance of the formulation according to the present invention, are given below. These compounds, apart from obviously CBD, can be defined as "cannabimimetics" and behave in a "cannabinoidergic" manner, that is, they are able to interact with CB1 and CB2 receptors. The formulation according to the present invention is able to achieve the desired effects even if CBD is completely absent in the same formulation.

Terpenes and other cannabinoid-like components work as minor cannabinoids by powerfully modulating receptors, ionotropic channels, and enzymes associated with the cannabinoid system and show therapeutic potential singly or synergistically with other phytocannabinoids. Basically, in the plant, nature is concerned with producing different molecules that act in agonist and antagonist ways on different receptors, as primary and secondary metabolites of the plant itself.

Cannabidiol (CBD) is one of the 142 phytocannabinoids, which are the substances identified in cannabis sativa. CBD does not produce the same psychotropic effects as THC; in fact, if administered simultaneously, it may attenuate some of them. CBD would enhance the analgesic efficacy of THC by prolonging its duration of action (by activating the serotonergic pathway at the dorsal raphe level) and at the same time reduce its side effects on heart rate, respiration, and body temperature. Specifically, CBD interacts as an antagonist toward GPR55 receptors, toward vanilloid receptors TRPV1 and TRPV2, and as an agonist toward serotonin 5-HT1a receptors.

Cannabidiol has a low affinity for the cannabinoid receptors CB1 and CB2 but acts as an indirect antagonist of these receptors. It may potentiate some of the effects of THC by increasing CB1 receptor density or through another mechanism related to the CB1 receptor. Cannabidiol inhibits cytochrome P450, and the enzymes CYP3A and CYP2C.

Cannabidiol may also act as an antagonist of GPR55, a G-receptor-coupled protein, a cannabinoid receptor expressed in the caudate nucleus and putamen of the brain. It has also been observed to act as a partial 5-HT1 receptor; this interaction may be associated with the antidepressant, anxiolytic, and neuroprotective effects of cannabidiol. It is also a positive allosteric modulator of µ-opioid and δ-opioid receptors.The pharmacological effects of cannabidiol have also been attributed to PPAR-γ agonism and intracellular calcium release.

Research suggests that CBD may exert some of its pharmacological actions through the inhibition of Fatty Acid Amide Hydrolase (FAAH), the enzyme responsible for the hydrolysis of anandamide, one of the main endocannabinoids produced by the body. It has also been hypothesized that some of the metabolites of CBD may have effects that contribute to the biological activity of CBD itself. Additional receptors for this molecule are CB1, CB2 and vaniloids and appear to be involved in neuromodulating chronic pain disorders. Recently, cannabidiol was identified as a novel inverse agonist of GPR12 similar to the GPR55 family.

Cannabidiol has also shown potential activity in the treatment of neurodegenerative diseases, and particularly with regard to Parkinson's disease and multiple sclerosis; in the case of Parkinson's, its activity is attributable to the neuromodulation contributed by CBD, while in the case of multiple sclerosis, the most impactful feature is related to its anti-inflammatory ability.

The skin is a sensory organ and therefore has dense innervation. The sensory nerve endings are of different types. There are dilatated endings: these are the lanceolate endings and Merkel-Ranvier discs, which come in contact with Merkel cells, endocrine cells in the epidermis. Corpuscular endings are located in the most sensitive areas: cutaneomucosal, Ruffini's, Meissner's, Vater-Pacini's or Golgi's corpuscles. Free endings are fine unmyelinated branches of myelinated fibers and are found in the dermis or epidermis, excluding the stratum corneum. The neurovegetative fibers terminate around vessels, hair erector muscles, and sweat glands. Skin innervation is so dense and subtle that neurocutaneous, cell-to-cell connections could be described as synapses. Neuromediators are chemicals that mediate nerve information. About 30 of them have been found in the skin. Nerve growth factors intervene a little upstream and control not only neuronal growth but also the release of neurotransmitters. The best known is NGF or "nerve growth factor." The nervous system can modulate all skin functions by changing the properties of the cells after activation by neuromodulators of their specific receptors, which are generally coupled to the G protein; and here comes back the G protein-bound receptor where CBD interacts.

Capsaicin is a chemical compound found, in various concentrations, in plants of the genus Capsicum (e.g., in hot chili peppers). Capsaicin is used as an analgesic in topical ointments and transdermal patches to relieve pain, in concentrations ranging from 0.025% to 0.1%. It can be applied as a cream, often in combination with other rubefacients, to provide temporary relief of mild muscle and joint pain associated with arthritis, back pain, strains, and sprains. Indeed, the receptors that are activated by this substance are inactivated after an initial intense activation. This is accompanied by a depletion in the content of a lipid, PIP2, at the level of the cell membrane, which results in a desensitization of the nerve ending toward the stimulus that initially excited it. This is why preparations containing capsaicin are used against muscle and rheumatic pain.

Capsaicin is also used to reduce the symptoms of peripheral neuropathies, such as post-herpetic neuralgia caused by shingles. Capsaicin creams have been used to treat itching caused by psoriasis, but several clinical studies have concluded that there is insufficient evidence to attribute capsaicin an anti-itching effect.

Piperine is an alkaloid found in black pepper; the spicy taste of capsaicin and piperine is due to activation of heat- and acid-sensitive TRPV1 ion channels in nociceptors (pain-sensitive nerve cells). Piperine was also found to inhibit human CYP3A4 and P-glycoprotein enzymes, which are important for the metabolism and transport of xenobiotics and metabolites. In animal studies, piperine has also been seen to inhibit other enzymes important in drug metabolism. By inhibiting drug metabolism, piperine can increase the bioavailability of various compounds.

Palmitoylethanolamide, or PEA, is the amide of an endogenous fatty acid and belongs to the class of nuclear factor agonists. PEA binds to specific nuclear receptors and performs a wide variety of biological functions related to chronic pain and inflammation. it is thought to be primarily targeted by PPAR-α. In the current state of knowledge, PEA is considered a dietary supplement with anti-inflammatory and pain-relieving properties.

Nerolidol (3,7,11-trimethyl-1,6,10-Dodecatrien-3-ol) is a natural, highly lipophilic sesquiterpene alcohol that is found in various essential oil plants. Chemically, nerolidol exists in two geometric isomers, a trans and a cis form. The use of nerolidol is widespread in various fields. It has been widely used in cosmetics (e.g., shampoos and perfumes) and in non-cosmetic products (e.g., detergents and cleaners). In fact, U.S. Food and Drug Administration (FDA) has also allowed the use of nerolidol as a food flavoring agent. The idea of using only food-grade terpenes allows for safer topical actives. The range of pharmacological and biological activities of nerolidol, that include antioxidant, anti-microbial, anti-biofilm, anti-parasite, insecticide, anti-ulcer, skin penetration stimulator, anti-tumor, anti-nociceptive, and anti-inflammatory. Since chemical compounds belonging to the sesquiterpenes group are well known for their antioxidant properties, the same properties were investigated for nerolidol. Nerolidol has been shown to have potent antioxidant properties in counteracting the effect of ROS by protecting cells from oxidative damage to lipids, proteins, and DNA. Studies have shown that nerolidol exhibited potent antimicrobial activity against Staphylococcus aureus FDA 209P, 14 methicillin-susceptible S. aureus (MSSA) strains, and 20 methicillin-resistant S. aureus (MRSA) strains. Nerolidol possesses antibacterial activity against various strains of Staphylococcus aureus, including MRSA, by disrupting cell membranes as indicated by the increased loss of K+ ions from bacterial cells. The observed effects could be due to the presence of the long aliphatic chain in the chemical structure of nerolidol.

Many bacteria are known to possess the ability to produce biofilm, which is defined as a community of microorganisms held together by a self-produced extracellular matrix attached to living or inert surfaces such as polystyrene, glass, stainless steel, and blood components in different environments. Because of the complexity of biofilm structure formation, microbial biofilms pose a significant challenge to the medical and pharmaceutical industries. Biofilm formation induces microbial resistance to antimicrobial agents as well as to the body's immune system. In addition, it has been associated with increased resistance to antibiotics, leading to biofilm-associated infections that complicate the treatment procedure. Nerolidol has shown anti-biofilm activity against several pathogens. The anti-biofilm activity was attributed to the presence of cis- and trans-nerolidol in the essential oil of C. odorata. Cis-nerolidol at 0.01% (v/v) has been shown to inhibit S. aureus biofilm formation by more than 80%, while trans-nerolidol at a similar concentration exerts an inhibition of 45%.

Various antifungal agents have been developed to control the spread of fungal diseases such as candidiasis. However, there are serious questions about the safety of these drugs because of their known side effects and the possible development of antifungal drug resistance. Therefore, the focus has shifted to bioprospecting natural products to overcome or control fungal infections. In fact, essential oils have been extensively studied and shown to be effective against fungal infections.

Gastric ulcer affects thousands of people worldwide and is known to be caused by an imbalance between aggressive (acid, pepsin) and protective (secretion and action of mucus and bicarbonate) factor in the stomach. It is induced by several factors, such as stress, smoking, nutritional deficiencies and ingestion of nonsteroidal anti-inflammatory drugs (NSAIDs). In the stress-induced ulceration model, treatment with nerolidol at 50, 250 and 500 mg/k caused a significant reduction in ulcerative lesion index (ULI) of 41.22, 51.31 and 56.57, respectively, compared with animals in the control group.

Transdermal penetration has gained much attention as an alternative route to intravenous and oral drug delivery systems. However, the application of transdermal delivery is limited by poor drug permeability, as the stratum corneum acts as a rate-limiting lipophilic barrier against the absorption of chemical and biological agents. As a result, terpenes are often used as skin penetration promoters due to their wide range of physical-chemical properties such as low skin irritability and good toxicological profile as well as adsorption-enhancing ability. A more than 20-fold increase in diffusion rate for transdermal penetration of several drugs, particularly 5-fluorouracil, has been found with the use of nerolidol. This high ability to enhance permeation has been attributed to the structure of nerolidol, which is suitable for alignment within the lipid lamellae of the stratum corneum in order to disrupt stratum corneum organization.

In human studies, administration of 4% nerolidol (unspecified isomer) in a preliminary test for a maximization study with single occlusive application for 48 h did not cause skin irritation. On the other hand, application of undiluted nerolidol (unspecified isomer) to intact and abraded skin caused well-defined erythema or mild edema in rabbits after 48 h, while 5% nerolidol in diethyl phthalate caused very mild edema in one animal and was eliminated after 48 h. Regarding skin sensitization, human studies showed that nerolidol (unspecified isomer) (4%) did not cause positive reactions in maximization tests or repeated insult tests. In animal studies, administration of nerolidol in guinea pigs caused weak reactions in two adjuvant tests with concentrations of 3% and 10%. However, no cross-sensitization was observed when guinea pigs induced with farnesyl acetate were sensitized with nerolidol.

Myrcene (β-myrcene) is an abundant monoterpene that is the main constituent in many plant species, including hops and cannabis. It is a well-known flavoring agent and aroma (food additive) used in food and beverage production. The main reported biological properties of β-myrcene-are anxiolytic, antioxidant, anti-aging, anti-inflammatory, and analgesic. Myrcene (7-methyl-3-methylene-1,6-octadiene) is a popular food additive used as a flavoring agent in food and beverage production. It is further used in consumer products such as cosmetics, soaps and detergents. In addition to its use in a variety of consumer products, β-Myrcene is used as a starting material for commercially important fragrances and flavors such as menthol, nerol, geraniol and linalool.

β-myrcene found within the cannabis plant possesses anti-inflammatory, analgesic, and sedative activities, which adds to the effects of classic phytocannabinoids and may generate synergistic interactions. β-myrcene may act synergistically with tetrahydrocannabinol and other cannabinoids, such as CBD, to enhance the activity of cannabis and ultimately increase its psychoactive potential. However, a recent study suggested that the functional effects of cannabis-derived terpenoids have not been detected, either alone or in combination with Δ9-tetrahydrocannabinol and cannabidiol. Therefore, their ability to produce entourage activity from direct effects at cannabinoid receptors cannot be fully determined. The study concluded that none of the tested terpenes present in the cannabis plant (β-myrcene, pinene, caryophyllene and limonene) have direct interaction with CB1 or CB2 receptors. In addition, there were unaltered changes in Δ9-tetrahydrocannabinol and cannabidiol activity. This study agrees with a previous study by Santiago and his colleague, in which they rule out direct activation of CB1 or CB2 or modulate signaling of the phytocannabinoid agonist Δ9-tetrahydrocannabinol by β-myrcene in the cannabis plant. However, both studies cannot rule out the existence of an entourage effect of myrcene because they examined only cannabinoids that signal through a pathway. There are possibilities of emergent entourage effects through the impact of terpenoids on other pathways of the endocannabinoid system or through non-cannabinoid receptor mechanisms that are important for the behavioral effect of cannabis strains.

β-Caryophyllene is a naturally occurring bicyclic sesquiterpene found in many essential oils. One cyclobutane ring is present in the molecule, a rarity in natural organic substances. Also notable is the fact that the other 9-membered fused ring has a double bond in a trans configuration.

The anti-inflammatory activity of this terpene was identified by popular medicine long before its molecular identification. In the past 10 years we have only skimmed the surface of the vast therapeutic potential of this harmless and fragrant scent.

In 2008 in Zurich, an international team of scientists discovered that β-caryophyllene is the first direct activator of CB2 (Cannabinoid Receptor 2) that can be taken with food, and that its anti-inflammatory action is mediated precisely by selective binding to this receptor.

Borneol is a bicyclic organic compound belonging to the terpene family. In this compound the hydroxyl group is arranged in the endo position. Isoborneol is the corresponding eso isomer; it is easily oxidized into the corresponding ketone, camphor. One of its historical names is "camphor of Borneo," from which it gets its present name.

Borneol is effective in the prevention and treatment of nerve injury in ischemic stroke. Its mechanisms of action include improvement of cerebral blood flow, inhibition of neuronal excitotoxicity, blockade of Ca²⁺ overload, and resistance to reactive oxygen species injury in the acute ischemic phase. In the subacute ischemic phase, Borneol is able to intervene in inflammatory reactions and prevent excessive neuronal death. In the advanced phase, Borneol promotes neurogenesis and angiogenesis in the treatment of ischemic stroke. Because the neuroprotective effects of Borneol are mediated by various factors, it promotes the passage of other drugs across the blood-brain barrier to exert synergistic therapeutic effects.

Incorporation of permeation enhancers is one of the most widely used approaches for drug delivery across biological membranes. Permeation enhancers help deliver drugs across various physiological barriers such as cerebral capillary endothelium, stratum corneum, corneal epithelium, and mucous membranes that pose resistance to the entry of most drugs. Borneol has been incorporated predominantly as an adjuvant in traditional Chinese formulations of centrally acting drugs to improve the bioavailability of drugs to the brain. Alteration in cell membrane lipid structures and modulation of multiple triphosphate and adenosine binding transporters as well as splicing proteins are major factors to the blood-brain barrier opening functions of Borneol. Because of these mechanisms of altering membrane properties, this compound has also shown promising potential to enhance drug penetration through other barriers as a promoter of permeation across the blood-brain barrier, mucosal barriers including nasal and gastrointestinal linings, transcorneal, transdermal.

α-pinene is a bicyclic monoterpene and the most widely distributed terpenoid in nature. It appears in conifers and countless plant essential oils, with an insect-repellent role. The molecule has an anti-inflammatory effect via PGE-1 (Gil et al., 1989) and is a bronchodilator in humans at low levels of exposure (Falk et al., 1990). Pinene is an important component of the essential oils of Sideritis (Kose et al., 2010) and Salvia (Ozek et al., 2010), having important activity against MRSA (Methicillin-resistant Staphylococcus). α-Pinene, then, forms the biosynthetic basis for CB2 ligands, such as HU-308 (Hanus et al., 1999).

In addition to this, it appears to be a broad-spectrum antibiotic (Nissen et al., 2010), and this property was probed through essential oils extracted from three legal varieties of hemp precisely: Futura, Fibranova, and Carmagnola. The results showed that EOs from industrial hemp can significantly inhibit microbial growth, to varying degrees depending on variety and planting time.

Honokiol and magnolol are structurally similar to cannabinoids but are derived from non-cannabis plants; they were initially identified as components of the genus Magnolia. In the 1990s, they were shown to have anti-free radical and lipid peroxidation inhibitory activities. Subsequently, honokiol, magnolol have shown antioxidant, anti-inflammatory, antimicrobial, anti-neurodegeneration, antidepressant, pain control, and liver protective properties.

The anti-inflammatory effects of magnolol and honokiol are mediated through inhibition of the MEKK-1 downstream pathway in NF-kappaB activation signaling. They show antinociceptive action on inflammatory and glutamatergic pain. These two compounds block inflammatory pain induced by glutamate, substance P and PGE2 with similar potency and efficacy. They are able to inhibit the release of inflammatory cytokines by inhibition of Ca²⁺-permeable TRPV3 receptors in human epidermal keratinocytes. Furthermore, in one study magnolol was shown to be a partial CBD receptor agonist, (EC50 = 3.28 µM) with selectivity for CB2 subtype, while honokiol was less potent showing full agonistic activity at CB1 and antagonistic properties at CB2. They have ability to interact and integrate in modulation of the cutaneous vanilloid and endocannabinoid systems, while not originating from cannabis.

Obviously, they can be used alternatively or in combination with CBD and/or acetaminophen. Honokiol and magnolol are fat-soluble with LogP over 4 and therefore poorly bioavailable. This explains the need for a delivery system such as SEDDS, especially an oil formulation because they have different stabilities depending on the pH of the formulation.

Paracetamol is one of the best known and most widely used analgesic and antipyretic drugs in the world, available without prescription in both single- and multi-component preparations. Previously, paracetamol was believed to induce analgesia by inhibiting cyclooxygenase enzymes. Recently, it has been shown that the main analgesic mechanism of acetaminophen is its metabolization to N-acyl phenolamine (AM404), which acts on vanilloid TRPV1 and cannabinoid CB1 receptors in the brain and C-fiber terminals in the spinal dorsal horn.

Therefore, paracetamol induces analgesia by acting not only on the brain but also on the spinal cord. Paracetamol is a well-tolerated drug and produces few gastrointestinal side effects. Our interest lies in its synergistic activity with terpenes and other possible actives on TRPV1 and CB1 receptors. Paracetamol inhibits COX like NSAIDs but has no anti-inflammatory activity. Cyclooxygenases, particularly prostaglandin H synthase (PGHS), are responsible for the synthesis of prostaglandins and thromboxanes. COXs are characterized by two catalytic subunits, operating one with cyclooxygenase and the other with peroxidase function, respectively.

Paracetamol, compared with NSAIDs, inhibits the peroxidase subunit, preventing the peroxidation of PGG2 to PGH2. Within the peroxidase site of COX, acetaminophen acts as a reducing factor. Although acetaminophen is able to inhibit cyclooxygenases, unlike NSAIDs, it lacks anti-inflammatory activity. In fact, in peripheral tissues and, especially, during inflammation, the high concentration of endogenous hydroperoxides, produced by inflammatory cells, oxidize the Fe radical at the peroxidase site, preventing the action of acetaminophen.

This action mechanism explains why paracetamol cannot counteract inflammation and has only an antipyretic and analgesic action at the central level. On the other hand, the absence of peripheral COX inhibition does not induce gastric and renal side effects typical of NSAIDs. For this reason, it is used little or not at all topically. But by taking advantage of the bioavailability provided by SEDDS, it can work as a modulator of the cutaneous endocannabinoid system.

It should be considered that the skin has 10% of the metabolic capacity of the liver. Therefore, the activity of acetaminophen and its metabolites at the level of the cutaneous endocannabinoid system has been considered. Understanding skin metabolism is important when considering an active and its effective dose.

Several studies have identified the activity of skin enzymes such as cytochromes P450, flavin monooxygenase, glutathione-S-transferase, N-acetyltransferase, and sulfotransferase (in vitro, ex vivo, and biopsy). The viable epidermis is known as the most enzymatically active part of the skin. Whereas the enzymatic activity of the dermis is much weaker than that of the epidermis.

Phosphatidylcholines (PCs) are a class of phospholipids that incorporate choline as their head group. They are a major component of biological membranes and can be easily obtained from several readily available sources, such as egg yolk or soy, from which they are extracted mechanically or chemically with hexane. They are part of the lecithin group, brownish-yellow fatty substances found in animal and plant tissues. Dipalmitoylphosphatidylcholine (lecithin) is a major constituent of lung surfactant and is often used in the lecithin-sphingomyelin ratio to calculate fetal lung maturity. Phosphatidylcholines are present in all plant and animal cells, but are absent in the membranes of most bacteria, including Escherichia coli. Purified phosphatidylcholine is commercially produced.

Endocannabinoids are synthesized from phospholipids (mainly phosphatidylethanolamine, phosphatidylcholine, and phosphatidylinositol) located in the cell membrane. As a result of the transfer of arachidonic acid from phosphatidylcholine to phosphatidylethanolamine, N-arachidonoyl phosphatidylethanolamine is formed, which is hydrolyzed to AEA by phospholipases D, C and A2. However, 2-AG is formed during the hydrolysis of phosphatidylinositol, which is mainly catalyzed by DAGL.

An example of preparation and some examples of application in therapeutic and cosmetic use of the formulation according to the present invention are given below.

### Example of preparation

Caprylic/capric triglyceride, Isopropyl myristate, Isopropyl palmitate, Tocopherol, Lecithin, Ascorbyl palmitate, Citric acid are weighed and mixed. Then synthetically produced Cannabidiol is added under slow stirring at room T°C. Once dissolution is achieved, Olea europaea fruit oil, Persea gratissima oil, C12-15 alkyl benzoate, Linum usitatissimum seed oil are added in a closed container. As soon as the mass is homogeneous, Propylene glycol caprylate, Apricot kernel oil PEG-6 esters, Polysorbate 80 are added at 20°-25°C under slow stirring. When the mass is homogeneous, Limonene, Eucalyptus globulus oil, Pinene, Camphor, Terpineol, Terpinolene, Methyl eugenol, Ethoxydiglycol, Borneol, Isoborneol are introduced into the mixture in a closed environment in the order as above and should be mixed until completely dissolved. Add under slow stirring always under temperature control: Beta-caryophyllene, Myrcene, Nerolidol.

A table of the preferred composition ranges of a formulation made according to the preparation described above is also given:

| Nome | Range % |
|---|---|
| Polysorbate 80 | 16-23 |
| Isopropyl myristate | 5-13 |
| Olea europaea fruit oil | 4.9-18 |
| Caprylic/capric triglyceride | 5-18 |
| Propylene glycol caprylate | 4-16 |
| Isopropyl palmitate | 2.4-13 |
| Limonene | 5-13 |
| Apricot kernel oil PEG-6 esters | 3-13 |
| Persea gratissima oil | 3-7 |
| C12-15 alkyl benzoate | 1-5 |
| Ethoxydiglycol | 0-2.6 |
| Borneol | 0-5 |
| Isoborneol | 0-5 |
| Eucalyptus globulus oil | 0-10 |
| Linum usitatissimum seed oil | 0.5-3 |
| Cannabidiol - synthetically produced | 0-6 |
| Beta-caryophyllene | 0-3 |
| Tocopherol | 0.01-2 |
| Lecithin | 0-0.5 |
| Ascorbyl palmitate | 0-0.5 |
| Citric acid | 0-0.5 |

### Application example 1 - therapeutic use

Product applicable on intact skin even following surgery with well-established scar. After examining the patient and identifying the pathology for which the patient presents (fibromyalgia, painful scar, joint pain from peripheral nerve injury, cutaneous allodynia, somatic pain point), the point(s) to be treated is determined. The formulation includes as the lipophilic portion a mixture of natural oils including high oleic olive oil, avocado oil, linseed oil, with medium chain triglycerides derived from coconut, in a percentage ranging from 5 to 60 percent; as the amphiphilic portion a mixture of surfactants comprising polysorbate, oleyl polyoxyglycerides, and with the possible presence of the coemulsifier propylene glycol monocaprylate, in concentrations varying from 5 to 45% depending on the concentration and Log P of the active or mixture of actives; as penetration promoters: limonene and eucalyptol, in concentrations ranging from 1 to 20 percent. The actives in the formulation include cannabidiol from 1 to 2 % ; β-caryophyllene from 0.1 to 35 %; myrcene from 0.5 to 10 %. This is done by applying 3 drops to each spot and, by digito-pressure, massaging for a few seconds. The oil thus formulated will overcome the cutaneous horny barrier and continue its benefit for several hours. Warn the patient not to rub or wash the skin until 8 hours after application. This is the time required for SEDDS to pass the stratum corneum and bind to the endocannabinoid receptors of the cells.

### Application example 2 - therapeutic use

With the same method of application as in the previous example, a formulation was used that includes as the lipophilic portion a mixture of natural oils including high oleic olive oil and avocado oil, safflower oil, with medium chain triglycerides derived from coconut, in a percentage ranging from 5 to 40%; as the amphiphilic portion a mixture of surfactants polysorbate, oleyl polyoxyglycerides, and with the possible presence of the coemulsifier propylene glycol monocaprylate, in concentrations varying from 5 to 55% depending on the concentration and Log P of the active or mixture of the actives; as a penetration promoter: limonene in concentrations ranging from 1 to 20%; as actives cannabidiol from 1 to 3%; β-caryophyllene from 0.1 to 10%; myrcene from 0.5 to 10%.

### Application example 3 - therapeutic use

Using the same method of application as in the previous example, a formulation was used that includes the lipophilic portion comprising a mixture of natural oils including high oleic olive oil, avocado oil, and flaxseed oil, with medium chain triglycerides derived from coconut, in a percentage ranging from 5 to 40 percent; the amphiphilic portion comprising a mixture of surfactants including polysorbate, Caprilocaproyl polyoxyglycerides, and with the possible presence of the coemulsifier propylene glycol monocaprylate, in concentrations varying from 5 to 45% depending on the concentration and Log P of the active or mixture of actives; as penetration promoters: limonene and eucalyptus in concentrations ranging from 3 to 20%; as actives cannabidiol from 2 to 10%; nerolidol from 0.5 to 35%; borneol from 2 to 35% ; PEA from 0.1 to 5%.

### Application example 4 - therapeutic use

Using the same application method as in the previous example, a formulation was used that includes as the lipophilic portion a mixture of natural oils including high oleic olive oil, avocado oil, and linseed oil, with medium chain triglycerides derived from coconut, in a percentage ranging from 5 to 50%; as the amphiphilic portion, a mixture of surfactants including polysorbate, Lauroyl polyoxyglycerides, and with the possible presence as a coemulsifier of propylene glycol monocaprylate, in concentrations varying from 5 to 55% depending on the concentration and Log P of the active or mixture of actives; as penetration promoters: limonene and eucalyptus in concentrations ranging from 3 to 30%; as actives: capsaicin 0.4 to 10% ; piperine 0.1% to 35%; alfapinene 0.1% to 35%; PEA 0.1 to 35%.

### Application example 5 - therapeutic use

Patient to be treated for different pathologies or to be rehabilitated. Product has topical use only: identify the criticality to be resolved (trauma, spinal pathology, rehabilitation of a limb, muscle-tensive headache).

Identify the exit course of the peripheral nerve closest to the site to be treated and then apply 5 drops along the course without rubbing but proceed with digito-pressure. Carry out the treatment according to rehabilitation techniques. Can also be used with the aid of an application device.

The formulation includes as a lipophilic portion a mixture of natural oils including high oleic olive oil, avocado oil, linseed oil, with medium chain triglycerides derived from coconut, in a percentage ranging from 5 to 60 percent; as the amphiphilic portion a mixture of surfactants comprising polysorbate, oleyl polyoxyglycerides, and with the possible presence of the coemulsifier propylene glycol monocaprylate, in concentrations varying from 5 to 45% depending on the concentration and Log P of the active or mixture of actives; as penetration promoters: limonene and eucalyptol, in concentrations ranging from 1 to 20 %. The actives in the formulation include cannabidiol from 1 to 2 percent; β-caryophyllene from 0.1 to 35 %; myrcene from 0.5 to 10 %.

### Application example 6 - therapeutic use

Using the same application method as in the previous example, a formulation was used that includes the lipophilic portion comprising a mixture of natural oils including high oleic olive oil, avocado oil, and linseed oil, with medium chain triglycerides derived from coconut, in a percentage ranging from 5 to 40 %; the amphiphilic portion comprising a mixture of surfactants including polysorbate, Caprilocaproyl polyoxyglycerides, and with the possible presence of the co-emulsifier propylene glycol monocaprylate, in concentrations varying from 5 to 45% depending on the concentration and Log P of the active or mixture of actives; as penetration promoters: limonene and eucalyptus in concentrations ranging from 3 to 20%; as actives cannabidiol from 2 to 10%; nerolidol from 0.5 to 35%; borneol from 2 to 35%; PEA from 0.1 to 5%.

### Application example 7 - therapeutic use

Using the same application method as in the previous example, a formulation was used that includes as the lipophilic portion a mixture of natural oils including high oleic olive oil, avocado oil, and linseed oil, with medium chain triglycerides derived from coconut, in a percentage ranging from 5 to 50%; as the amphiphilic portion, a mixture of surfactants including polysorbate, Lauroyl polyoxyglycerides, and with the possible presence as a coemulsifier of propylene glycol monocaprylate, in concentrations varying from 5 to 55% depending on the concentration and Log P of the active or mixture of actives; as penetration promoters: limonene and eucalyptus in concentrations ranging from 3 to 30%; as actives: capsaicin from 0.4 to 10%; piperine from 0.1% to 35%; alfapinene from 0.1% to 35%; PEA from 0.1 to 35%.

### Application example 8 - cosmetic use.

Formulation to be used topically only. Applicable to perform functional skin and muscle recovery treatments. Use the necessary drops on the treatment spot (never more than 5) and start the chosen treatment. Applicable also for positions forced to the headrest so as to give more comfort to the person. Before applying the product, thoroughly cleanse the chosen spots. Then proceed with a circular finger motion to improve localized absorption. Since it is a skin conditioner, with defense barrier passage, the person should have washed beforehand and should maintain the product for at least 8 hours.

The formulation includes as lipophilic portion a mixture of natural oils including high oleic olive oil, avocado oil, linseed oil, with medium chain triglycerides derived from coconut, in a percentage ranging from 5 to 60 percent; as the amphiphilic portion a mixture of surfactants comprising polysorbate, oleyl polyoxyglycerides, and with the possible presence of the coemulsifier propylene glycol monocaprylate, in concentrations varying from 5 to 45% depending on the concentration and Log P of the active or mixture of actives; as penetration promoters: limonene and eucalyptol, in concentrations ranging from 1 to 20 percent. The actives include: cannabidiol from 1 to 2%; β-caryophyllene from 0.1% to 35%; myrcene from 0.5 to 10%.

### Application example 9 - cosmetic use

Using the same application method as in the previous example, a formulation was used that includes as the lipophilic portion a mixture of natural oils including high oleic olive oil and avocado oil, safflower oil, with medium-chain triglycerides derived from coconut, in a percentage ranging from 5 to 40 percent; as the amphiphilic portion a mixture of surfactants polysorbate, oleyl polyoxyglycerides, and with the possible presence of the coemulsifier propylene glycol monocaprylate, in concentrations varying from 5 to 55% depending on the concentration and Log P of the active or mixture of the actives; as a penetration promoter: limonene in concentrations ranging from 1 to 20%; actives include cannabidiol from 1% to 3%; β-caryophyllene from 0.1% to 10%; myrcene from 0.5% to 10%.

### Application example 10 - veterinary therapeutic use.

Product applicable on intact, uninjured skin. The indications are completely superimposable on those for medical use. Shave the hair in order to facilitate the absorption of SEDDS at the stratum corneum level. The amount of drops will be determined by the surface to be treated (1 to 20 gtt). There is no need to scrub, so as to limit the time of application by the operator.

The treatment can be continued at the pet's home. Possible ingestion by the pet produces no side effects due to the particular composition of sedds that can be used orally.

The formulation includes as lipophilic portion a mixture of natural oils including high oleic olive oil, avocado oil, linseed oil, with medium chain triglycerides derived from coconut, in a percentage ranging from 5 to 60 percent; as the amphiphilic portion a mixture of surfactants comprising polysorbate, oleyl polyoxyglycerides, and with the possible presence of the coemulsifier propylene glycol monocaprylate, in concentrations varying from 5 to 45% depending on the concentration and Log P of the active or mixture of actives; as penetration promoters: limonene and eucalyptol, in concentrations ranging from 1 to 20 percent. Active substances include cannabidiol from 1% to 2%; β-caryophyllene from 0.1% to 35%; myrcene from 0.5% to 10%.

### Application example 11 - veterinary therapeutic use.

With the same method of application as in the previous example, a formulation was used that includes as the lipophilic portion a mixture of natural oils including high oleic olive oil and avocado oil, safflower oil, with medium chain triglycerides derived from coconut, in a percentage ranging from 5 to 40%; as the amphiphilic portion a mixture of surfactants polysorbate, oleyl polyoxyglycerides, and with the possible presence of the coemulsifier propylene glycol monocaprylate, in concentrations varying from 5 to 55% depending on the concentration and Log P of the active or mixture of the actives; as a penetration promoter: limonene in concentrations ranging from 1 to 20%; actives include cannabidiol from 1% to 3%; β-caryophyllene from 0.1% to 10%; myrcene from 0.5 to 10%.

### Application example 12 - veterinary therapeutic use

Using the same method of application as in the previous example, a formulation was used that includes the lipophilic portion comprising a mixture of natural oils including high oleic olive oil, avocado oil, and linseed oil, with medium chain triglycerides derived from coconut, in a percentage ranging from 5 to 40 percent; the amphiphilic portion comprising a mixture of surfactants including polysorbate, Caprilocaproyl polyoxyglycerides, and with the possible presence of the coemulsifier propylene glycol monocaprylate, in concentrations varying from 5 to 45% depending on the concentration and Log P of the active or mixture of actives; as penetration promoters: limonene and eucalyptus in concentrations ranging from 3 to 20%; as actives cannabidiol from 2% to 10%; nerolidol from 0.5% to 35%; borneol from 2 to 35%; PEA from 0.1% to 5%.

## Claims

1. Self-emulsifying formulation for skin use, comprising a highly lipophilic active component capable of interacting with cannabinoid and vanilloid receptors, a lipophilic portion, an amphiphilic portion, and a skin penetration-promoting portion derived from essential oils.

2. Formulation according to claim 1, in which the active component comprises a substance chosen from the group consisting of cannabidiol, capsaicin, piperine, palmitoylethanolamide, nerolidol, myrcene, β-caryophyllene, borneol, α-pinene, honokiol, magnolol, acetaminophen, phosphatidylcholine, or a mixture of two or more of said substances.

3. Formulation according to claim 2, in which the active component is comprised in the formulation in a percentage between 0.1% and 35%.

4. Formulation according to any one of the preceding claims 1 to 3, in which the lipophilic portion comprises unsaturated fatty acids, and preferably oleic acid and/or linoleic acid, in combination with medium-chain triglycerides.

5. Formulation according to any one of the preceding claims 1 to 4, in which the lipophilic portion additionally comprises saturated fatty acid esters, such as isopropylpalmitate and isopropylmyristate.

6. Formulation according to any one of the preceding claims 1 to 5, in which the lipophilic portion comprises benzoic acid esters with a C12-C15 alcohol, and tocopherol.

7. Formulation according to any one of the preceding claims 1 to 6, in which the amphiphilic portion comprises nonionic surfactants, such as polysorbates, and in particular polysorbate 80, in combination with co-surfactants such as oleylpolyoxyglycerides, ethoxydiglycol, and propylene glycol monocaprylate.

8. Formulation according to any one of the preceding claims 1 to 7, in which the skin penetration-promoting portion derived from the essential oils comprises eucalyptus oil, and in particular includes eucalyptol; other terpenes preferred are: limonene linalyl acetate, bisabolol.

9. Formulation according to any one of the preceding claims 1 to 8, wherein said formulation comprises as a lipophilic portion a mixture of natural oils including high oleic olive oil, avocado oil, linseed oil, with medium chain triglycerides derived from coconut, in a percentage ranging from 5 to 60%; as the amphiphilic portion a mixture of surfactants comprising polysorbate, oleyl polyoxyglycerides, and with the possible presence of the coemulsifier propylene glycol monocaprylate, in concentrations varying from 5 to 45% depending on the concentration and Log P of the active or mixture of actives; as penetration promoters: limonene and eucalyptol, in concentrations ranging from 1 to 20%.

10. Formulation according to any one of the preceding claims 1 to 8, wherein said formulation comprises as lipophilic portion a mixture of natural oils including high oleic olive oil and avocado oil, safflower oil, with medium chain triglycerides derived from coconut, in a percentage ranging from 5 to 40%; as the amphiphilic portion a mixture of surfactants polysorbate, oleyl polyoxyglycerides, and with the possible presence of the coemulsifier propylene glycol monocaprylate, in concentrations varying from 5 to 55% depending on the concentration and Log P of the active or mixture of the actives; as a penetration promoter: limonene in concentrations ranging from 1 to 20%.

11. Formulation according to any one of the preceding claims 1 to 8, wherein said formulation comprises the lipophilic portion comprising a mixture of natural oils including high oleic olive oil, avocado oil, and linseed oil, with medium chain triglycerides derived from coconut, in a percentage ranging from 5 to 40%; the amphiphilic portion comprising a mixture of surfactants including polysorbate, Caprilocaproyl polyoxyglycerides, and with the possible presence of the coemulsifier propylene glycol monocaprylate, in concentrations varying from 5 to 45% depending on the concentration and Log P of the active or mixture of actives; as penetration promoters: limonene and eucalyptol in concentrations ranging from 3 to 20%.

12. Formulation according to any one of the preceding claims 1 to 8, wherein said formulation comprises as a lipophilic portion a mixture of natural oils including high oleic olive oil, avocado oil, and linseed oil, with medium chain triglycerides derived from coconut, in a percentage ranging from 5 to 50%; as the amphiphilic portion, a mixture of surfactants including polysorbate, Lauroyl polyoxyglycerides, and with the possible presence as a coemulsifier of propylene glycol monocaprylate, in concentrations varying from 5 to 55% depending on the concentration and Log P of the active or mixture of actives; as penetration promoters: limonene and eucalyptol in concentrations ranging from 3 to 30%.

13. Therapeutic use of the formulation according to any of the preceding claims 1 to 12, for the treatment of the human or animal body.

14. Therapeutic use according to claim 13, for the treatment of conditions such as fibromyalgia, painful scar, joint pain from peripheral nerve injury, cutaneous allodynia, somatic pain point.

15. Cosmetic use of the formulation according to any of the preceding claims 1 to 12, for the treatment of the human or animal body.
